# EUROPEAN PATENT SPECIFICATION

(11) **EP 0 831 835 B1**
(45) Date of publication and mention of the grant of the patent: **30.10.2002**
(21) Application number: 96920403.1
(22) Date of filing: 23.05.1996
(51) Int. Cl.: A61K 31/55, A61P 43/00

(54) **TREATING ANOREXIA**
BEHANDLUNG DER ANOREXIE
TRAITEMENT DE L'ANOREXIE

(30) Priority: 01.06.1995 US 457249
(43) Date of publication of application: 01.04.1998
(73) Proprietor: ELI LILLY AND COMPANY, Indianapolis, Indiana 46285 (US)
(72) Inventor: BEASLEY, Charles, M., Jr., Indianapolis, IN 46256 (US)
(74) Representative: Pritchard, Judith
(86) International application number: US9607467
(87) International publication number: WO96038152

(56) References cited:
- WO-A-94/22871
- US-A- 5 229 382
- US-A- 5 234 945
- US-A- 5 250 571
- US-A- 5 250 572
- US-A- 5 352 686
- MOORE N A ET AL: "THE BEHAVIORAL PHARMACOLOGY OF OLANZAPINE, A NOVEL ATYPICAL ANTIPHSYCHOTIC AGENT" JOURNAL OF PHARMACOLOGY AND EXPERIMENTAL THERAPEUTICS,US,AMERICAN SOCIETY FOR PHARMACOLOGY AND, vol. 262, no. 2, page 545-551 XP000573947 ISSN: 0022-3565
- MELTZER H.Y. ET AL: "Recent Advances in the Pharmacotherapy of Schizophrenia" ACTA PSYCHIATRICA SCANDINAVIA, SUPPL., vol. 90, no. 384, 1994, pages 95-101, XP002125196 Denmark
- KENNEDY S.H. ET AL: "Current Perspectives on drug Therapies for Anorexia Nervosa and Bulimia Nervosa" DRUGS, vol. 41, no. 3, 1991, pages 367-377, XP002125197
- CROW S.J. ET AL: "Rational Therapy of Eating Disorders" DRUGS, vol. 48, no. 3, 1994, pages 372-379, XP002125198
- Neuropsychopharmacology (1996) 14 (2) pp. 87-95

## Description

This invention provides a method for using 2-methyl-4-(4-methyl-1-piperazinyl)-10H-thieno[2,3-b][1,5] benzodiazepine in the manufacture of a medicament, for the treatment of a clinically significant decrease in appetite.

The present invention relates to the field of food intake modifiers, and more particularly to the field of modifiers that can be administered to increase or decrease human food intake.

The physiology of the control of food intake is not well understood. Many cogent theories have been advanced based on data and observation. Several of these theories are discussed in "Physiology of the Control of Food Intake", Kissileff *et al.*, Ann. Rev. Nutr. 2:371-418 (1982); Russek "Current Status of the Hepatostatic Theory of Food Intake Control", Appetite 2:137-143 (1981); and Friedman *et al.*, "The Physiological Psychology of Hunger: A Physiological Perspective", Physiological Review, 83(6):409-431 (1976). Notwithstanding the current knowledge in this area, the effect that the administration of any given substance will have upon a mammal's food intake is normally difficult if not impossible to predict in the absence of significant food intake data stemming from prior experience with that compound or substance.

J.Pharmacol Exp.Ther (1992, 262, pp.545-551 describes olanzapine as an antipsychotic agent with 5-hydroxytryptamine and dopamine D₁/D₂ antagonist properties.

Acta Pychiatrica Scandinavica, Supplement (1994),90(384),pp.95-101 describes potent 5-HT₂ antagonist with DA antagonistic effects, including olanzapine.

Drugs (1991), 41(3), pp. 367-377 describes various drug therapies for anorexia nervosa and bulimia nervosa. Lorazepam and Oxazepam are suggested as treatments for refeeding anorexic patients.

Drugs (1994), 48(3), pp.372-379 describes studies on seratonin antagonists and antipsychotic agents for the treatment of anorexia nervosa. The antipsychotic agents showed no significant difference compared to the placbo.

WO94/22871 describes certain thieno-indole compounds being 5HT2c and 5HT2b antagonists. The compounds are said to be useful in treating various disorders, including anorexia.

It is known that the compound 2-methyl-4-(4-methyl-1-piperazinyl)-10H-thieno[2,3-b][1,5] benzodiazepine can provide antipsychotic activity and is less likely to induce extrapyramidal symptoms. However, Applicant has discovered that surprisingly 2-methyl-4-(4-methyl-1-piperazinyl)-10H-thieno[2,3-b][1,5] benzodiazepine can be useful for treating clinically significant decreases in appetite. The compound 2-methyl-4-(4-methyl-1-piperazinyl)-10H-thieno[2,3-b][1,5] benzodiazepine is known and described in U.S. Patent No. 5,229,382.

The presently claimed invention provides a method in the manufacture of a medicament for treating a clinically significant decrease in appetite comprising administering an effective amount of 2-methyl-4-(4-methyl-1-piperazinyl)-10H-thieno[2,3-b][1,5] benzodiazepine or a pharmaceutically acceptable salt thereof to a human patient in need of such treatment.

The 2-methyl-4-(4-methyl-1-piperazinyl)-10H-thieno[2,3-b][1,5]benzodiazepine compound is of the formula 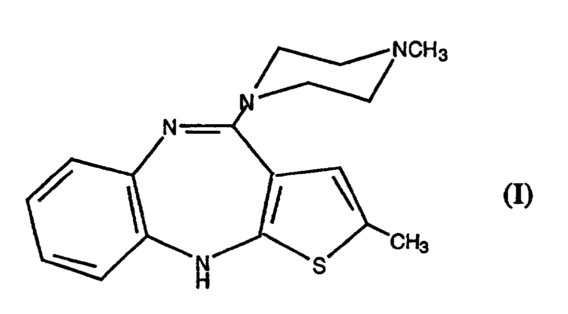 or an acid addition salt thereof. The free base of formula (I) is 2-methyl-4-(4-methyl-1-piperazinyl)-10H-thieno[2,3-b][1,5]benzodiazepine.

The substantially pure crystalline anhydrous Form II 2-methyl-4-(4-methyl-1-piperazinyl)-10H-thieno[2,3-b][1,5] benzodiazepine (Form II) has a typical X-ray powder diffraction pattern substantially as follows, using a Sieman's D5000 diffractometer equipped with a copper radiation source, wherein d represents the interplaner spacing:

| **d** | **I/I**_{**1**} |
|---|---|
| 10.2689 | 100.00 |
| 8.577 | 7.96 |
| 7.4721 | 1.41 |
| 7.125 | 6.50 |
| 6.1459 | 3.12 |
| 6.071 | 5.12 |
| 5.4849 | 0.52 |
| 5.2181 | 6.86 |
| 5.1251 | 2.47 |
| 4.9874 | 7.41 |
| 4.7665 | 4.03 |
| 4.7158 | 6.80 |
| 4.4787 | 14.72 |
| 4.3307 | 1.48 |
| 4.2294 | 23.19 |
| 4.141 | 11.28 |
| 3.9873 | 9.01 |
| 3.7206 | 14.04 |
| 3.5645 | 2.27 |
| 3.5366 | 4.85 |
| 3.3828 | 3.47 |
| 3.2516 | 1.25 |
| 3.134 | 0.81 |
| 3.0848 | 0.45 |
| 3.0638 | 1.34 |
| 3.0111 | 3.51 |
| 2.8739 | 0.79 |
| 2.8102 | 1.47 |
| 2.7217 | 0.20 |
| 2.6432 | 1.26 |
| 2.6007 | 0.77 |

Form I 2-methyl-4-(4-methyl-1-piperazinyl)-10H-thieno[2,3-b][1,5] benzodiazepine (Form I) has a typical X-ray powder diffraction pattern substantially as follows, using a Sieman's D5000 diffractometer equipped with a copper radiation source, wherein d represents the interplaner spacing:

| **d** | **I/I**_{**1**} |
|---|---|
| 9.9463 | 100.00 |
| 8.5579 | 15.18 |
| 8.2445 | 1.96 |
| 6.8862 | 14.73 |
| 6.3787 | 4.25 |
| 6.2439 | 5.21 |
| 5.5895 | 1.10 |
| 5.3055 | 0.95 |
| 4.9815 | 6.14 |
| 4.8333 | 68.37 |
| 4.7255 | 21.88 |
| 4.6286 | 3.82 |
| 4.533 | 17.83 |
| 4.4624 | 5.02 |
| 4.2915 | 9.19 |
| 4.2346 | 18.88 |
| 4.0855 | 17.29 |
| 3.8254 | 6.49 |
| 3.7489 | 10.64 |
| 3.6983 | 14.65 |
| 3.5817 | 3.04 |
| 3.5064 | 9.23 |
| 3.3392 | 4.67 |
| 3.2806 | 1.96 |
| 3.2138 | 2.52 |
| 3.1118 | 4.81 |
| 3.0507 | 1.96 |
| 2.948 | 2.40 |
| 2.8172 | 2.89 |
| 2.7589 | 2.27 |
| 2.6597 | 1.86 |
| 2.6336 | 1.10 |
| 2.5956 | 1.73 |

The x-ray powder diffraction patterns set forth herein were obtained with a copper K of wavelength = 1.541A. The interplanar spacings in the column marked "d" are in Angstroms. The typical relative intensities are in the column marked "I/I₁". The detector was a Kevex silicon lithium solid state detector.

As used herein "substantially pure" shall refer to anhydrous Form II associated with about < 5% Form I; and most preferably it shall refer to about < 2% Form I. It is further preferred that "substantially pure" shall refer to < 0.5% related substances.

As used herein, the term "2-methyl-4-(4-methyl-1-piperazinyl)-10H-thieno[2,3-b][1,5]benzodiazepine" refers to a technical grade of 2-methyl-4-(4-methyl-1-piperazinyl)-10H-thieno(2,3-b][1,5]benzodiazepine when no specific solvate or polymorph is named. Typically, the technical grade 2-methyl-4-(4-methyl-1-piperazinyl)-10H-thieno[2,3-b][1,5] benzodiazepine contains less than about 5% undesired related substances and may be a mixed polymorph. Such technical grade 2-methyl-4-(4-methyl-1-piperazinyl)-10H-thieno[2,3-b][1,5] benzodiazepine may contain less than about 1% undesired related substances.

As used herein, the term "treating" includes prophylaxis of the named condition or amelioration or elimination of the condition once it has been established. Most preferably, the term refers to amelioration or elimination of the clinically significant decrease in appetite.

As used herein, the term "clinically significant decrease in appetite" shall include conditions characterized by disturbed sense of body image, marked weight loss, morbid fear of obesity, and amenorrhea in women. The term shall include, but is not limited to anorexia nervosa, bulimia nervosa, and general anorexia. It is particularly preferred that the term refers to anorexia nervosa. The term further includes, but is not limited to Restricted type anorexia nervosa and binge-eating/purging type anorexia nervosa.

It is further preferred that the term "clinically significant decrease in appetite" refers to general anorexia. The term "general anorexia" refers to a clinically significant loss of appetite which may be characterized by clinically significant weight loss resulting from suboptimal or inadequate appetite for the consumption of food. The term general anorexia refers to a condition in which the patient refuses or lacks sufficient appetite to maintain a minimally normal body weight.

As used herein "minimally normal level" or "minimally normal level weight" shall refer to a weight which is at least about 85% of the weight that is considered normal for that person's age and height. The normal weight may be computed using published version of the Metropolitan Life Insurance tables or pediatric growth charts. The term "minimally normal level weight" may be determined using the individuals body mass index (BMI), (as weight in kilograms/height in meters²). A BMI of at least about 17.5kg/m² is considered a minimally normal level weight.

Onset of such eating disorders is typically at adolescence, occasionally prepubertal, and less commonly in adulthood. Long term follow up studies of patients diagnosed with anorexia nervosa have reported mortality rates of 10 to 20%.

The typical patient suffering from anorexia nervosa is preoccupied with food. Such patients often study diets, calories, hoard, conceal, and waste food. The essential feature of anorexia nervosa is that the patient refuses to maintain a minimally normal body weight, is intensely afraid of gaining weight, and exhibits a significant disturbance in the perception of shape or size of his or her body.

The individual suffering from a clinically significant decrease in appetite maintains a body weight that is below a minimally normal level for age and height. During childhood or early adolescence, there may be a failure to make expected weight gains rather than a weight loss.

Usually weight loss is accomplished primarily through reduction in total food intake. Although individuals may begin excluding from their diet what they perceive to be high calorie foods, most eventually end up with a very restricted diet that may include only a few foods. Additional methods of weight loss include purging or excessive exercise.

The experience and significance of body size and shape are distorted in patients suffering from anorexia nervosa.

The results of pharmacological studies show that 2-methyl-4-(4-methyl-1-piperazinyl)-10H-thieno[2,3-b][1,5]benzodiazepine has muscarinic cholinergic receptor activity. The compound is active at the dopamine D-1 and D-2 receptors as indicated by an IC50 of less than 1 uM in the 3H-SCH233390 (Billard, et al. Life Sciences **35**:1885 (1984)) and the 3H spiperone (Seeman et al Nature **216**:717 (1976)) binding assays respectively. Further, olanzapine is active at the 5-HT-2 receptor and 5-HT1C receptor. The complex pharmacological profile of the compound provides a medicament which can be useful for the treatment of a clinically significant decrease in appetite.

The usefulness of the compound for treating various anorexias can be supported by the following studies as described.

### I. Rodent Study.

Male Sprague Dawley CD rats (Charles River, Wilmington, Mass.) are individually housed in cages with ad libitum access to Animal Chow and tap water. Temperature is maintained at approximately 21° C. Rats are adapted to vivarium conditions for at least a week before tests begin.

At the start of the experiments, the rats are weighed. Rats receive ascending doses of 2-methyl-4-(4-methyl-1-piperazinyl)-10H-thieno[2,3-b][1,5]benzodiazepine (2, 7.5, 15 and 20 mg/kg) by intragastric intubation (2 ml/kg). Each of the four doses has its own isotonic saline control intubation, given in counterbalanced order, so that on any test half the rats receive saline and the other half receive 2-methyl-4-(4-methyl-1-piperazinyl)-10H-thieno[2,3-b][1,5]benzodiazepine. Tests are conducted every 3-4 days. Food intake (± 0.1 g) is recorded hourly for the 5 h after intubation and after 24 h.

Results are analyzed by analysis of variance for each cumulative time period, with 2-methyl-4-(4-methyl-1-piperazinyl)-10H-thieno[2,3-b][1,5]benzodiazepine (saline v. 2-methyl-4-(4-methyl-1-piperazinyl)-10H-thieno[2,3-b][1,5]benzodiazepine) and Dose (2, 7.5, 15 and 20 mg/kg mg/kg) as factors.

Surprisingly, animal studies using rodents and dogs suggested that 2-methyl-4-(4-methyl-1-piperazinyl)-10H-thieno[2,3-b][1,5]benzodiazepine would not be effective for the treatment of clinically significant decrease in appetite in these animals. In fact, many such animals lost weight with 2-methyl-4-(4-methyl-1-piperazinyl)-10H-thieno[2,3-b][1,5]benzodiazepine administration.

Unexpectedly, Applicants discovered that 2-methyl-4-(4-methyl-1-piperazinyl)-10H-thieno[2,3-b][1,5]benzodiazepine can be useful for the treatment of clinically significant loss of appetite in humans!

### II. Clinical observations.

A double-blind multicenter clinical trial was designed to assess the safety and efficacy of 2-methyl-4-(4-methyl-1-piperazinyl)-10H-thieno[2,3-b][1,5] benzodiazepine in human patients. Patients were randomized to 2-methyl-4-(4-methyl-1-piperazinyl)-10H-thieno[2,3-b][1,5]benzodiazepine or placebo. The results of the study suggest that 2-methyl-4-(4-methyl-1-piperazinyl)-10H-thieno[2,3-b][1,5] benzodiazepine can be useful for the treatment of anorexia.

Another double blind multicenter clinical trial was completed using 1,996 human subjects. The subjects were randomized to 2-methyl-4-(4-methyl-1-piperazinyl)-10H-thieno[2,3-b][1,5]benzodiazepine or haloperidol. The study continued for 6 weeks, with possible extensions. The 2-methyl-4-(4-methyl-1-piperazinyl)-10H-thieno[2,3-b][1,5]benzodiazepine compound was administered at 5-20 mg/day, while haloperidol subjects received 5-20 mg haloperidol/day. The results of this study showed that 2-methyl-4-(4-methyl-1-piperazinyl)-10H-thieno[2,3-b][1,5]benzodiazepine treated subjects exhibited a statistically significant dose dependent weight gain and an increase in appetite. The results of this study provide further support that 2-methyl-4-(4-methyl-1-piperazinyl)-10H-thieno[2,3-b][1,5]benzodiazepine can be useful for the treatment of clinically significant anorexia.

The results of these clinical studies provide support that administration of a pharmaceutically effective amount of 2-methyl-4-(4-methyl-1-piperazinyl)-10H-thieno[2,3-b][1,5]benzodiazepine may benefit human subjects suffering from or susceptible to clinically significant loss of appetite.

The compound 2-methyl-4-(4-methyl-1-piperazinyl)-10H-thieno[2,3-b][1,5]benzodiazepine can be used for the methods of this invention, both in its free base and acid addition salt forms. The acid addition salts are preferably the pharmaceutically acceptable, non-toxic addition salts with suitable acids, such as those of inorganic acids, for example hydrochloric, hydrobromic, nitric, sulfuric or phosphoric acids, or of organic acids, such as organic carboxylic acids, for example glycollic, maleic, hydroxymaleic, fumaric, malic, tartaric, citric or lactic acid, or organic sulfonic acids for example methane sulfonic, ethane sulfonic, 2-hydroxyethane sulfonic, toluene-p-sulfonic or naphthalene-2-sulfonic acid.

The compound has an IC₅₀ of less than 1 mM in the ³H-QNB binding assay described by Yamamura, HI and Snyder, SH in Proc.Nat.Acad.Sci. USA 71 1725 (1974) indicating that it has muscarinic-cholinergic activity.

The 2-methyl-4-(4-methyl-1-piperazinyl)-10H-thieno-[2,3-b][1,5]benzodiazepine compound is effective over a wide dosage range, the actual dose administered being dependent on the condition being treated. For example, in the treatment of adult humans, dosages of from about 0.25 to 50 mg, preferably from 1 to 30 mg, and most preferably 1 to 20 mg per day may be used. A once a day dosage is normally sufficient, although divided doses may be administered. For treatment of cognitive dysfunction, a dose range of from 1 to 30 mg, preferably 1 to 20 mg per day is suitable. Radiolabelled 2-methyl-4-(4-methyl-1-piperazinyl)-10H-thieno-[2,3-b][1,5]benzodiazepine, can be detected in the saliva and thus the compound can potentially be monitored in patients to assess compliance.

A preferred formulation of the invention is a solid oral formulation comprising from about 1 to about 20 mg or 1 to 10 mg of active 2-methyl-4-(4-methyl-1-piperazinyl)-10H-thieno[2,3-b][1,5] benzodiazepine as an effective amount of the active ingredient.

Most preferably, the solid oral formulation is contained in packaging materials which protect the formulation from moisture and light. For example, suitable packaging materials include amber colored high density polyethylene bottles, amber colored glass bottles, and other containers made of a material which inhibits the passage of light. Most preferably, the packaging will include a desiccant pack. The container may be sealed with an aluminum foil blister to provide the desired protection and maintain product stability.

The 2-methyl-4-(4-methyl-1-piperazinyl)-10H-thieno-[2,3-b][1,5]benzodiazepine compound will normally be administered orally or by injection and, for this purpose, it is usually employed in the form of a pharmaceutical composition.

Accordingly, pharmaceutical compositions comprising 2-methyl-4-(4-methyl-1-piperazinyl)-10H-thieno[2,3-b][1,5]benzodiazepine, as active ingredient associated with a pharmaceutically acceptable carrier may be prepared. In making the compositions of the invention conventional techniques for the preparation of pharmaceutical compositions may be used. For example, the active ingredient will usually be mixed with a carrier, or diluted by a carrier, or enclosed within a carrier which may be in the form of a capsule, sachet, paper or other container. When the carrier serves as a diluent, it may be solid, semi-solid or liquid material which acts as a vehicle, excipient or medium for the active ingredient. The active ingredient can be adsorbed on a granular solid container for example in a sachet. Some examples of suitable carriers are lactose, dextrose, sucrose, sorbitol, mannitol, starches, gum acacia, calcium phosphate, alginates, tragacanth, gelatin, syrup, methyl cellulose, methyl- and propyl-hydroxy-benzoate, talc, magnesium stearate or mineral oil. The compositions of the invention may, if desired, be formulated so as to provide quick, sustained or delayed release of the active ingredient after administration to the patient. For example, one such preferred quick release formulation is described in U.S. Patent Nos. 5,079,018, 5,039,540, 4,305,502, 4,758,598, and 4,371,516, hereby incorporated by reference. Such formulation most preferably comprises 2-methyl-4-(4-methyl-1-piperazinyl)-10H-thieno[2,3-b][1,5] benzodiazepine, water, hydrolyzed gelatin, and mannitol.

Depending on the method of administration, the compositions for the treatment of central nervous system conditions may be formulated as tablets, capsules, injection solutions for parenteral use, gel or suspension for transdermal delivery, suspensions or elixirs for oral use or suppositories. Preferably the compositions are formulated in a unit dosage form, each dosage containing from 0.25 to 100 mg, more usually 1 to 30 mg, of the active ingredient. When a sustained release formulation is desired, the unit dosage form may contain from 0.25 to 200 mg of the active ingredient. A preferred formulation of the invention is a capsule or tablet comprising 0.25 to 75 mg or 1 to 30 mg of active ingredient together with a pharmaceutically acceptable carrier therefor. A further preferred formulation is an injection which in unit dosage form comprises 0.25 to 30 mg or 1 to 30 mg of active ingredient together with a pharmaceutically acceptable diluent therefor.

The materials for the present invention can be purchased or prepared by a variety of procedures well known to those of ordinary skill in the art. The 2-methyl-4-(4-methyl-1-piperazinyl)-10H-thieno[2,3-b][1,5] benzodiazepine compound can be prepared as described by Chakrabarti in U.S. Patent No 5,229,382 ('382), herein incorporated by reference in its entirety.

Compound characterization methods include, for example, x-ray powder pattern analysis, thermogravimetric analysis (TGA), differential scanning calorimetery (DSC), titrametric analysis for water, and H¹-NMR analysis for solvent content.

The following examples are provided for purposes of illustration and are not to be construed as limiting the scope of the claimed invention.

### Preparation 1

### Form II 2-methyl-4-(4-methyl-l-piperazinyl)-10H-thieno[2,3-b][1,5] benzodiazepine

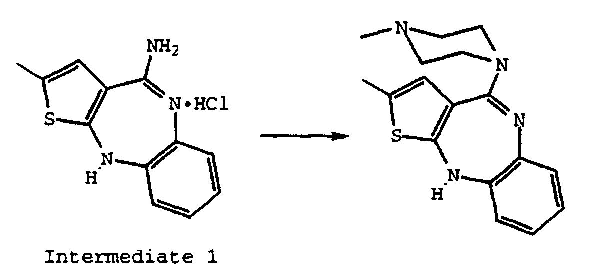

In a suitable three neck flask the following was added:

| | |
|---|---|
| Dimethylsulfoxide (analytical) | 6 volumes |
| Intermediate 1 | 75 g |
| N-Methylpiperazine (reagent) | 6 equivalents |

Intermediate 1 can be prepared using methods known to the skilled artisan. For example, the preparation of the Intermediate 1 is taught in the '382 patent.
A sub-surface nitrogen sparge line was added to remove the ammonia formed during the reaction. The reaction was heated to 120°C and maintained throughout the duration of the reaction. The reactions were followed by HPLC until ≤ 5% of the intermediate 1 was left unreacted. After the reaction was complete, the mixture was allowed to cool slowly to 20°C (about 2 hours). Each reaction mixture was then transferred to an appropriate three neck round bottom flask and water bath. To this solution with agitation was added 10 volumes reagent grade methanol and the reaction was stirred at 20°C for 30 minutes. Three volumes of water was added slowly over about 30 minutes. The reaction slurry was cooled to zero to 5°C and stirred for 30 minutes. The product was filtered and the wet cake was washed with chilled methanol. The wet cake was dried in vacuo at 45°C overnight. The product was identified as technical 2-methyl-4-(4-methyl-1-piperazinyl)-10H-thieno[2,3-b][1,5] benzodiazepine.
Yield: 76.7%; Potency: 98.1%

A 270 g sample of technical 2-methyl-4-(4-methyl-1-piperazinyl)-10H-thieno[2,3-b][1,5] benzodiazepine was suspended in ethyl acetate (2.7 L). The mixture was heated to about 76°C and maintained at about 76°C for about 30 minutes. The mixture was allowed to cool to about 25°C. The resulting product was isolated using vacuum filtration. The product was identified as Form II 2-methyl-4-(4-methyl-1-piperazinyl)-10H-thieno[2,3-b][1,5] benzodiazepine using x-ray powder analysis.
Yield: 197 g.

### EXAMPLE 1

A portion of the hydroxypropyl cellulose was dissolved in purified water to form a solution for granulation. The remaining hydroxypropyl cellulose (total of 4.0% w/w final tablet weight), which was an extra fine grade, was combined with the 2-methyl-4-(4-methyl-1-piperazinyl)-10H-thieno[2,3-b][1.5] benzodiazepine (1.18% w/w), lactose (79.32% w/w) and a portion of the crospovidone (5% w/w) in a high shear granulator. All ingredients were security sieved prior to addition and dry blended in the granulator. This mixture was then granulated with the hydroxypropyl cellulose solution in the high shear granulator. The granulation was wet sized using standard methods. The wet granulation was then dried in a fluidized bed dryer and sized. The material was then added to a tumble bin mixer.
The running powders consisting of microcrystalline cellulose (granular) (10% w/w), magnesium stearate (0.5% w/w), and the remainder of the crospovidone were added to the sized granulation. The mixture was blended and compressed with the appropriate tooling on tablet compression equipment.

### Subcoating:

Hydroxypropyl methylcellulose (10% w/w) was mixed with purified water to form a solution. Core tablets were divided into approximately equal sections and spray coated with the hydroxypropyl methylcellulose solution . The operation was performed in a perforated coating pan.

### Coating of Core Tablets:

Color Mixture White (hydroxypropyl methylcellulose, polyethylene glycol, polysorbate 80, and titanium dioxide) was mixed with purified water to form the coating suspension. Subcoated tablets were divided into approximately equal sections and spray coated with the coating suspension described above. The operation was performed in a perforated coating pan.
The coated tablets were lightly dusted with carnauba wax and imprinted with appropriate identification.

### EXAMPLE 2

The process substantially as described above in Example 1 was repeated using the following ingredients to provide pharmaceutically elegant tablet formulations containing 1, 2.5, 5, 7.5, and 10 mg 2-methyl-4-(4-methyl-1-piperazinyl)-10H-thieno[2,3-b][1,5] benzodiazepine, respectively, per tablet:

| 1 mg 2-methyl-4-(4-methyl-1-piperazinyl)-10H-thieno[2,3- bl[1,5]benzodiazepine per tablet: | |
|---|---|
| **Names of Ingredients** | **Quantity (mg/tablet)** |
| **Active Ingredient** | |
| 2-methyl-4-(4-methyl-1-piperazinyl)-10H-thieno[2,3-b][1,5]benzodiazepine | 1.0 |

| **Other Ingredients** | |
|---|---|
| Lactose | 67.43 |
| Hydroxypropyl Cellulose | 3.40 |
| Crospovidone | 4.25 |
| Microcrystalline Cellulose | 8.50 |
| Magnesium Stearate | 0.42 |

| **Subcoating** | |
|---|---|
| Hydroxypropyl Methylcellulose | 1.70 |

| **Coating** | |
|---|---|
| Color Mixture White | 3.47 |

| **Polishing** | |
|---|---|
| Carnauba Wax | trace |

| **Imprinting** | |
|---|---|
| Edible Blue Ink | trace |

| 2-methyl-4-(4-methyl-1-piperazinyl)-10H-thieno[2,3-b][1,5]benzodiazepine 2.5 mg Tablets | |
|---|---|
| **Names of Ingredients** | **Quantity (mg/tablet)** |
| **Active Ingredient** | |
| 2-methyl-4-(4-methyl-1-piperazinyl)-10H-thieno[2,3-b][1,5]benzodiazepine | 2.5 |

| **Other Ingredients** | |
|---|---|
| Lactose Hydroxypropyl Cellulose | 102.15 5.20 |
| Crospovidone | 6.50 |
| Microcrystalline Cellulose | 13.00 |
| Magnesium Stearate | 0.65 |

| **Subcoating** | |
|---|---|
| Hydroxypropyl Methylcellulose | 2.60 |

| **Coating** | |
|---|---|
| Color Mixture White | 5.30 |

| **Polishing** | |
|---|---|
| Carnauba Wax | trace |

| **Imprinting** | |
|---|---|
| Edible Blue Ink | trace |

| 2-methyl-4-(4-methyl-1-piperazinyl)-10H-thieno[2,3-b][1,5]benzodiazepine 5.0 mg Tablets | |
|---|---|
| **Names of Ingredients** | **Quantity (mg/tablet)** |
| **Active Ingredient** | |
| 2-methyl-4-(4-methyl-1-piperazinyl)-10H-thieno[2,3-b][1,5]benzodiazepine | 5.00 |

| **Other Ingredients** | |
|---|---|
| Lactose | 156.00 |
| Hydroxypropyl Cellulose | 8.00 |
| Crospovidone | 10.00 |
| Microcrystalline Cellulose | 20.00 |
| Magnesium Stearate | 1.00 |

| **Subcoating** | |
|---|---|
| Hydroxypropyl | 4.00 |
| Methylcellulose | |

| **Coating** | |
|---|---|
| Color Mixture White | 8.16 |

| **Polishing** | |
|---|---|
| Carnauba Wax | trace |

| **Imprinting** | |
|---|---|
| Edible Blue Ink | trace |

| 2-methyl-4-(4-methyl-1-piperazinyl)-10H-thieno[2,3-b][1,5]benzodiazepine 7,5 mg Tablets | |
|---|---|
| **Names of Ingredients** | **Quantity (mg/tablet)** |
| Active Ingredient | |
| 2-methyl-4-(4-methyl-1-piperazinyl)-10H-thieno[2,3-b][1,5]benzodiazepine | 7.50 |

| **Other Ingredients** | |
|---|---|
| Lactose | 234.00 |
| Hydroxypropyl Cellulose | 12.00 |
| Crospovidone | 15.00 |
| Microcrystalline Cellulose | 30.00 |
| Magnesium Stearate | 1.50 |

| **Subcoating** | |
|---|---|
| Hydroxypropyl Methylcellulose | 6.00 |

| **Coating** | |
|---|---|
| Color Mixture White | 12.24 |

| **Polishing** | |
|---|---|
| Carnauba Wax | trace |

| **Imprinting** | |
|---|---|
| Edible Blue Ink | trace |

| 2-methyl-4-(4-methyl-1-piperazinyl)-10H-thieno[2,3-b][1,5]benzodiazepine 10,0 mg Tablets | |
|---|---|
| **Names of Ingredients** | **Quantity (mg/tablet)** |
| **Active Ingredient** | |
| 2-methyl-4-(4-methyl-1-piperazinyl)-10H-thieno[2,3-b][1,5]benzodiazepine | 10.00 |

| **Other Ingredients** | |
|---|---|
| Lactose | 312.00 |
| Hydroxypropyl Cellulose | 16.00 |
| Crospovidone | 20.00 |
| Microcrystalline Cellulose | 40.00 |
| Magnesium Stearate | 2.00 |

| **Subcoating** | |
|---|---|
| Hydroxypropyl Methylcellulose | 8.00 |

| **Coating** | |
|---|---|
| Color Mixture White | 16.32 |

| **Polishing** | |
|---|---|
| Carnauba Wax | trace |

| **Imprinting** | |
|---|---|
| Edible Blue Ink | trace |

### EXAMPLE 4

### Pulvule Formulation

A pulvule formulation is prepared by blending the active with silicone starch, and filling it into hard gelatin capsules.

| | Per 300 mg capsule |
|---|---|
| Compound of the invention | 30.0 mg |
| Silicone | 2.9 mg |
| Starch flowable | 267.1 mg |

### EXAMPLE 5

### Tablet Formulation

A tablet formulation is made by granulating the active with appropriate diluent, lubricant, disintegrant and binder and compressing

| | |
|---|---|
| Compound of the invention | 10.0 mg |
| Magnesium stearate | 0.9 mg |
| Microcrystalline cellulose | 75.0 mg |
| Povidone | 15.0 mg |
| Starch, directly compressible | 204.1 mg |

### EXAMPLE 6

### Aqueous Injection Formulation

An aqueous injection of active is prepared as a freeze-dried plug, for reconstitution in a suitable, sterile diluent before use (to a total volume of 10 ml).

Compound of the invention is contacted with Mannitol N Hydrochloric acid and/or N sodium hydroxide to adjust pH to 5-5.5.

| | |
|---|---|
| Compound of the invention | 20.0 mg |
| Mannitol N Hydrochloric acid and/or N sodium hydroxide to adjust pH to 5-5.5. | 20.0 mg |

### EXAMPLE 7

### Controlled Release IM Formulation

A controlled release injection for intramuscular injection is formed from a sterile suspension of micronised active in an oleaginous vehicle.

| | |
|---|---|
| Compound of the invention | 50.0 mg |
| Aluminium stearate | 0.04 mg |
| Sesame oil | 2 ml |

### EXAMPLE 8

### Capsule Formulation

A formulation is prepared by blending the active with silicone starch and starch, and filling it into hard gelatine capsules.

| | Per 300 mg capsule |
|---|---|
| Compound of the invention | 2.5 mg |
| Starch flowable with 0.96% silicone 220 | 222.5 mg |
| Starch flowable | 75.0 mg |

### Example 9

### 2-methyl-4-(4-methyl-1-piperazinyl)-10H-thieno[2,3-b][1,5] benzodiazepine Granules

The granules were produced by blending the mannitol and Hydroxymethyl propyl cellulose in a high shear mixer; granulating with the aqueous suspension of 2-methyl-4-(4-methyl-1-piperazinyl)-10H-thieno[2,3-b][1,5] benzodiazepine and polysorbate 20; wet sized and subsequently dried in a fluid bed dryer. These are dry sized and reblended prior to packaging.

| **1a. 250mg Sachets** | |
|---|---|
| **INGREDIENT** | **MG/SACHET** |
| **Active** | |
| 2-methyl-4-(4-methyl-1-piperazinyl)-10H-thieno[2,3-b][1,5]benzodiazepine | 2.50 |

| **Other Ingredients** | |
|---|---|
| Mannitol | 234.97 |
| Hydroxypropyl methyl cellulose 3cps | 12.50 |
| Polysorbate 20 | 0.028 |

| **1b. 750mg Sachets** | |
|---|---|
| **INGREDIENT** | **MG/SACHET** |
| **Active** | |
| 2-methyl-4-(4-methyl-1-piperazinyl)-10H-thieno[2,3-b][1,5]benzodiazepine | 7.50 |

| **Other Ingredients** | |
|---|---|
| Mannitol | 704.93 |
| Hydroxypropyl methyl cellulose 3cps | 37.49 |
| Polysorbate 20 | 0.08 |

| **1c. 1000mg Sachets** | |
|---|---|
| **INGREDIENT** | **MG/SACHET** |
| **Active** | |
| 2-methyl-4-(4-methyl-1-piperazinyl)-10H-thieno[2,3-b][1,5]benzodiazepine | 10.0 |

| **Other Ingredients** | |
|---|---|
| Mannitol | 939.90 |
| Hydroxypropyl methyl cellulose 3cps | 49.99 |
| Polysorbate 20 | 0.11 |

Such granules are most preferably contacted with an acidic medium if a suspension or solution is desired.

### Example 10

### Human Administration

A tablet formulation of 2-methyl-4-(4-methyl-1-piperazinyl)-10H-thieno[2,3-b][1,5]benzodiazepine, described supra., is adminstered to a human subject suffering from anorexia nervosa at a dosage rate of 20 mg/day.

## Claims

1. Use of 2-methyl-4-(4-methyl-1-piperazinyl)-10H-thieno[2,3-b][1,5] benzodiazepine, or a pharmaceutically acceptable salt thereof, in the manufacture of a medicament for the treatment of a clinically significant decrease in appetite in a human.

2. Use according to **Claim 1** wherein the clinically significant decrease in appetite is general anorexia.

3. Use according to **Claim 1** wherein 2-methyl-4-(4-methyl-1-piperazinyl)-10H-thieno[2,3-b][1,5] benzodiazepine is substantially pure Form II 2-methyl-4-(4-methyl-1-piperazinyl)-10H-thieno[2,3-b][1,5] benzodiazepine having a typical X-ray powder diffraction pattern as follows, using a Sieman's D5000 diffractometer wherein d represents the interplaner spacing:
| **d** |
|---|
| 10.2689 |
| 8.577 |
| 7.4721 |
| 7.125 |
| 6.1459 |
| 6.071 |
| 5.4849 |
| 5.2181 |
| 5.1251 |
| 4.9874 |
| 4.7665 |
| 4.7158 |
| 4.4787 |
| 4.3307 |
| 4.2294 |
| 4.141 |
| 3.9873 |
| 3.7206 |
| 3.5645 |
| 3.5366 |
| 3.3828 |
| 3.2516 |
| 3.134 |
| 3.0848 |
| 3.0638 |
| 3.0111 |
| 2.8739 |
| 2.8102 |
| 2.7217 |
| 2.6432 |
| 2.6007 |

4. Use according to **Claim 1 or 3** wherein the clinically significant decrease in appetite is anorexia nervosa.

5. Use according to **Claim 1 or 3** wherein the benzodiazepine is for administration in an effective amount of from 2 mg to 20 mg per day.

6. Use according to **Claim 1 or 3** wherein the clinically significant decrease in appetite is bulimia nervosa.

7. Use according to **Claim 1 or 3** wherein the diazepine is for administration as a pharmaceutically acceptable formulation.

8. Use according to **Claim 7** wherein the formulation is tablet.

9. Use according to **Claim 7** wherein the formulation is granule.

10. Use according to **Claim 7** wherein the formulation is a rapidly dissolving tablet.

## Patentansprüche

1. Verwendung von 2-Methyl-4-(4-methyl-1-piperazinyl)-10H-thieno[2,3-b][1,5]benzodiazepin oder eines pharmazeutisch annehmbaren Salzes hiervon zur Herstellung eines Arzneimittels zur Behandlung einer klinisch signifikanten Abnahme des Appetits bei einem Menschen.

2. Verwendung nach Anspruch 1, worin die klinisch signifikante Appetitabnahme allgemeine Anorexie ist.

3. Verwendung nach Anspruch 1, worin 2-Methyl-4-(4-methyl-1-piperazinyl)-10H-thieno[2,3-b][1.5]benzodiazepin im wesentlichen reine Form II von 2-Methyl-4-(4-methyl-1-piperazinyl)-10H-thieno[2,3-b][1,5]benzodiazepin mit einem typischen folgenden Röntgenbeugungsmuster am Pulver ist, das mittels eines Siemens D5000 Diffraktometers erhalten wurde, worin d für den Interplanarabstand steht:
| d | d (Fortsetzung) |
|---|---|
| 10,2689 | 4,141 |
| 8,577 | 3,9873 |
| 7,4721 | 3,7206 |
| 7,125 | 3,5645 |
| 6,1459 | 3,5366 |
| 6,071 | 3,3828 |
| 5,4849 | 3,2516 |
| 5,2181 | 3,134 |
| 5,1251 | 3,0848 |
| 4,9874 | 3,0638 |
| 4,7665 | 3,0111 |
| 4,7158 | 2,8739 |
| 4,4787 | 2,8102 |
| 4,3307 | 2,7217 |
| 4,2294 | 2,6432 |
| | 2,6007 |

4. Verwendung nach Anspruch 1 oder 3, worin die klinisch signifikante Appetitabnahme Anorexia nervosa ist.

5. Verwendung nach Anspruch 1 oder 3, worin das Benzodiazepin zur Verabreichung in einer wirksamen Menge von 2 mg bis 20 mg pro Tag vorgesehen ist.

6. Verwendung nach Anspruch 1 oder 3, worin die klinisch signifikante Appetitabnahme Bulimia nervosa ist.

7. Verwendung nach Anspruch 1 oder 3, worin das Diazepin zur Verabreichung als pharmazeutisch annehmbare Formulierung vorgesehen ist.

8. Verwendung nach Anspruch 7, worin die Formulierung eine Tablette ist.

9. Verwendung nach Anspruch 7, worin die Formulierung ein Granulat ist.

10. Verwendung nach Anspruch 7, worin die Formulierung eine schnell auflösende Tablette ist.

## Revendications

1. Utilisation de 2-méthyl-4-(4-méthyl-1-pipérazinyl)-10H-thiéno[2,3-b][1,5]benzodiazépine ou d'un sel pharmaceutiquement acceptable de celle-ci dans la préparation d'un médicament pour le traitement d'une diminution cliniquement significative de l'appétit chez un être humain.

2. Utilisation selon la revendication 1, dans laquelle la diminution cliniquement significative de l'appétit est l'anorexie générale.

3. Utilisation selon la revendication 1, dans laquelle la 2-méthyl-4-(4-méthyl-1-pipérazinyl)-10H-thiéno[2,3-b][1,5]benzodiazépine est de la 2-méthyl-4-(4-méthyl-1-pipérazinyl)-10H-thiéno[2,3-b][1,5]benzodiazépine de forme II substantiellement pure présentant un spectre de diffraction de rayons X sur des poudres caractéristique comme suit, obtenu en utilisant un diffractomètre Sieman D5000, dans lequel d représente l'espace entre les plans :
| d |
|---|
| 10,2689 |
| 8,577 |
| 7,4721 |
| 7,125 |
| 6,1459 |
| 6,071 |
| 5,4849 |
| 5,2181 |
| 5,1251 |
| 4,9874 |
| 4,7665 |
| 4,7158 |
| 4,4787 |
| 4,3307 |
| 4,2294 |
| 4,141 |
| 3,9873 |
| 3,7206 |
| 3,5645 |
| 3,5366 |
| 3,3828 |
| 3,2516 |
| 3,134 |
| 3,0848 |
| 3,0638 |
| 3,0111 |
| 2,8739 |
| 2,8102 |
| 2,7217 |
| 2,6432 |
| 2,6007 |

4. Utilisation selon la revendication 1 ou 3, dans laquelle la diminution cliniquement significative de l'appétit est l'anorexie nerveuse.

5. Utilisation selon la revendication 1 ou 3, dans laquelle la benzodiazépine est prévue pour une administration en une quantité efficace de 2 à 20 mg par jour.

6. Utilisation selon la revendication 1 ou 3, dans laquelle la diminution cliniquement significative de l'appétit est la boulimie nerveuse.

7. Utilisation selon la revendication 1 ou 3, dans laquelle la benzodiazépine est prévue pour une administration sous forme d'une formulation pharmaceutiquement acceptable.

8. Utilisation selon la revendication 7, dans laquelle la formulation est un cachet.

9. Utilisation selon la revendication 7, dans laquelle la formulation est un granule.

10. Utilisation selon la revendication 7, dans laquelle la formulation est un cachet à dissolution rapide.
